# EUROPEAN PATENT APPLICATION

(11) **EP 1 666 066 A1**
(43) Date of publication of application: **07.06.2006**
(21) Application number: 04773271.4
(22) Date of filing: 17.09.2004
(51) Int. Cl.: A61K 45/00, A61P 25/28, A61P 43/00, A61K 31/445

(54) **REMEDY FOR DOWN SYNDROME**

(30) Priority: 19.09.2003 JP 2003327393
(71) Applicant: Eisai Co., Ltd., Tokyo 112-8088 (JP)
(72) Inventor: KONDO, Tatsuro, Nishi-sonogi-gun, Nagasaki 851-2126 (JP)
(74) Representative: HOFFMANN EITLE
(86) International application number: PCT/JP2004/013636
(87) International publication number: WO 2005/027968

(57) **Abstract**

Because there is no fundamental treatment for Down's syndrome, and participation in society is the ultimate goal, the present invention provides a remedy and method for treating Down's syndrome that improve congenital delayed psychomotor development, and promote improvement in the performance of daily activities and socialization. The present invention is directed to a therapeutic agent for Down's syndrome that contains an acetylcholinesterase inhibitor, a therapeutic agent for improving mental retardation in Down's syndrome, and an agent for improving daily activities of patients. Specifically, the acetylcholinesterase inhibitor is donepezil hydrochloride. More specifically, the present invention is directed to a therapeutic agent for Down's syndrome, a therapeutic agent for improving mental retardation in Down's syndrome, and an agent for improving daily activities of patients with an IQ of 48 or lower and/or patients aged 36 or younger.

## Description

### Technical Field

The present invention relates to a novel medical application of donepezil or a pharmaceutically acceptable salt thereof, which is an acetylcholinesterase inhibitor, to Down's syndrome. More particularly, the present invention relates to a method for treating Down's syndrome by use of donepezil hydrochloride and a pharmaceutical composition containing donepezil hydrochloride for the treatment of Down's syndrome.

### Background Art

Down's syndrome is the most common disease caused by a chromosome aberration, and it mainly occurs as a result of an excess of chromosome 21, which is called 21 trisomy (not less than 90% of Down's syndrome cases are caused by standard trisomy 21). Down's syndrome occurs at a frequency of about 1 in 1000 individuals, and it is characterized by the fact that delayed psychomotor development can be seen after birth. In addition, Down's syndrome is often accompanied by various complications. For example, complications such as congential heart disease, gastrointestinal tract anomalies, infantile spasm, and pulmonitis and other infectious diseases frequently appear in infancy including the neonatal period; and obesity, acute paraplegia, alopecia areata, thyropathy, acute leukemia, refraction abnormalities of the eye, and exudative otitis media frequently appear in childhood through school age (see *Advances in Down syndrome and Rehabilitation - for improvement for Understanding,* edited by Valentine Dmitriev and Patricia Oelwein, translated by Kazuko Takei, 1st edition, Kyodo Isho Shuppan, 20 June 1992, pp. 1-12, 49-53, and 62-69). It is also known that the kinds of neuropathologies seen in Alzheimer's disease increase with age in adults with Down's syndrome. For example, amyloid-deposited senile plaques starting in the patient's thirties, and changes in neurofibrils starting in the patient's forties have been confirmed. Moreover, as the neuropathologies progress from the later thirties into the forties and beyond, the onset of Alzheimer-type dementia and behavorial changes due to dementia are also seen. It is presumed that 75% or more of the patients with Down's syndrome who are 60 and older will exhibit symptoms of Alzheimer's disease (see *Advances in Down syndrome and Rehabilitation - for improvement for Understanding,* cited above, "Relationship between Alzheimer-type dementia in Down's syndrome and apolipoprotein E phenotype" by Shuichi Ikeda and 5 others., Annual report of the Ministry of Health and Welfare Primary Amyloidisis Research Committee for 1995, 1996, p. 86-89).

Just as in the case of other congenital diseases, there is no fundamental method of treatment for Down's syndrome, so medical management subsequent to early discovery and diagnosis, as well as comprehensive rehabilitation involving pediatric care, education, training and the like is performed. In medical management the therapeutic techniques to treat complications such as infectious disease, congenital heart disease, gastrointestinal tract anomalies and the like have been developed. As a result, the survival rate and average age at death of Down's syndrome patients have markedly increased. In the field of rehabilitation, various rehabilitative programs have been developed to bring about a good relationship between the patient and society in general such as age-appropriate behavior, improvement in life skills and the like (see Nancy J Roizen, and one other. "Down's syndrome." The Lancet, 2003; 361(9365):1281-1289). With respect to the effectiveness of rehabilitation, however, it has been reported that an intelligence quotient (IQ) plays a major role, for example, in language functions in childhood, and sufficient learning effectiveness is not seen in patients with an IQ of 50 or less (see James H. Heller and 5 others. "Donepezil for the treatment of language deficits in adults with Down's syndrome: A preliminary 24-week open trial." American Journal of Medical Genetics 2003; 116(2):111-116).

In the midst of this therapeutic environment, pharmacotherapy has been investigated with the expectation that it will be effective when used together with rehabilitation and symptomatic treatments. For example, pharmacotherapy involving vitamins and 5-hydroxytryptophan have been investigated, but no efficacy was seen with respect to the development of motor skills, language skills, intelligence, or socialization (see *Advances in Down syndrome and Rehabilitation - for improvement for Understanding,* cited above). Moreover, recently the efficacy of piracetam, which is used in combination with antiepileptics and the like for the treatment of cortical myoclonus (spontaneously occurring involuntary muscle movements), for the treatment of Down's syndrome has been investigated. When piracetam was administered to a Down's syndrome model mouse, an effect in improving cognitive function was seen (see Timothy H. Moran and 5 others. 'The effect of piracetam on cognitive performance in a mouse model of Down's syndrome." Physiology & Behavior 2002; 77:403-409). However, in a double blind study of 25 human children with Down's syndrome, although an effect was seen on stimulation of the central nervous system such as aggressiveness, excitability, sexual arousal, restless sleep, and reduction in appetite, no improvement in cognitive function was seen (see Nancy J Roizen, and one other cited above).

On the other hand, donepezil hydrochloride is a drug with acetylcholinesterase inhibitory activity (see Japanese Patent No. 2578475). Therefore, at present, donepezil hydrochloride is used as a drug to inhibit the progression of dementia symptoms in mild to moderate Alzheimer-type dementia. In adults the initial dose is 3mg once a day to ameliorate adverse gastrointestinal effects, and after 1-2 weeks, the dose is increased to 5 mg once a day.

It is assumed that the onset of dementia will occur earlier and at a higher rate in patients with Down's syndrome than in healthy individuals. For example, it is known that the ratio of patients with neuropathological signs (for example, senile plaques) similar to those in Alzheimer's disease increases with advancing age even when behavioral symptoms that can be diagnosed as dementia are not present (see James B. Leverenz and one other. "Early amyloid deposition in the medical temporal lobe of young down syndrome patients: A regional quantitative analysis." Experimental Neurology, 150: 296-304). Therefore, the administration of donepezil hydrochloride to adult patients with Down's syndrome was attempted as pharmacotherapy. For example, an open study was conducted on 4 adult patients with Down's syndrome (age 64, IQ <20, dementia; age 38, IQ 53, symptoms of dementia; age 24, IQ 57, no symptoms of dementia; and age 27, IQ 58, no symptoms of dementia), and 5mg of donepezil hydrochloride was administered once a day for the first 6 weeks, followed by administration of 10mg once a day until the end of the study. In that study when a before-after comparison was made after 6 months of treatment with donepezil hydrochloride, a significant improvement in socialization and adaptive behavior was seen in patients with an IQ of 57 or higher who showed no symptoms of dementia, but no changes in daily life functions were seen (see Priya S Krishnani and 5 others. "Cholinergic therapy for Down's syndrome." Lancet, 1999; 353: 1064). In addition, a 24-week open study was conducted in which donepezil hydrochloride was administered to 6 adult patients with Down's syndrome to improve language function disorders. Among the plurality of language functions evaluated in that study, an improvement in the specified language function assessment test was seen only in language expression ability after 12 weeks of treatment in comparison with the initial part of the study (see James H. Heller, cited above). Moreover, with respect to the effect of IQ on drug efficacy, the results indicated a tendency for no effect to be seen in patients with an IQ of 50 or lower.

### Disclosure of the Invention

### Problems to be Solved by the Invention

Because there is no fundamental method of treatment for Down's syndrome, which is a congenital genetic disease, it is considered most important to improve the quality of life (QOL) of the patient and the patient's family by improving performance of daily activities, improving spontaneous and independent behavior, improving communication with the patient's surroundings, and the like. Therefore, the development of various rehabilitation programs and pharmacotherapies as approaches to treatment has been attempted. However, neither a rehabilitative program nor a medicine that exhibits sufficient efficacy has been found. There have been instances in which donepezil hydrochloride, which is a drug that inhibits the progression of Alzheimer-type dementia, has been used in patients with Down's syndrome who also exhibit symptoms of Alzheimer-type dementia and in groups of adult patients in which a large percentage exhibit neuropathological Alzheimer-like symptoms, but there have not been cases in which this drug has been administered for the purpose of improving the congenital delayed psychomotor development associated with Down's syndrome. In other words, it is necessary to establish a medicine or method of treatment that can improve mental retardation, and even promote mental development, as well as improve behavior in daily activities in patients up to their early thirties who exhibit almost no behavioral symptoms of Alzheimer-type dementia, and especially in patients younger than twenty, in which a large percentage do not show Alzheimer's symptoms either neuropathologically or neurochemically. Moreover, a medicine is strongly needed that can improve the congenital mental retardation or promote mental development in Down's syndrome patients with an IQ of 50 or less in whom rehabilitation cannot be expected to be effective.

Moreover, patients who develop symptoms of rapid retrogression are sometimes seen among pediatric patients with Down's syndrome. In this context, the term "symptoms of rapid retrogression" refers to a rapid retrogression in abilities of the daily lives and adaptive behavior. In most patients the onset occurs between puberty and adulthood, i.e., about twenty, but in early patients the onset occurs in the early teens. In aspects of movement and behavior, sudden slowness in performance of daily activities, paucity of expression, decrease in conversation, slumping posture, shuffling gait and the like are seen as specific symptoms. On the other hand, loss of interest, perseverative tendency and the like are seen in aspects of emotion and personality, and interpersonally an excess of nervous tension and inability to maintain interpersonal relationships are seen. Physically, sleep disorders, loss of appetite, weight loss, incontinence and the like may also occur. Thus, there is a strongly need that a medicine that can improve the symptoms of rapid retrogression in pediatric Down's syndrome.

### Means for Solving the Problems

As a result of diligent investigations into the above problems, the inventor has discovered that donepezil hydrochloride, a drug used to treat Alzheimer-type dementia, is effective as a drug to treat Down's syndrome, a congenital disease. In other words, the inventor has discovered that it is possible to improve mental retardation or even promote mental development in patients with Down's syndrome and also improve the everyday QOL in areas such as performance of daily activities, independent behavior, socialization, communication and the like. More specifically, the inventor has discovered that donepezil hydrochloride is effective in improving mental retardation or promoting mental development in patients with Down's syndrome who are 36 years old and younger, and it is also effective in patients with Down's syndrome who have an IQ of 50 or lower, a condition in which rehabilitation effect cannot be expected to be effective in improving mental retardation.

In other words, the present invention provides:
1. A therapeutic agent for Down's syndrome, comprising an acetylcholinesterase inhibitor,
2. The therapeutic agent for Down's syndrome according to item 1, wherein the acetylcholinesterase inhibitor is donepezil or a pharmaceutically acceptable salt thereof,
3. The therapeutic agent for Down's syndrome according to item 1 or 2, wherein an intelligence quotient of a patient with Down's syndrome is 48 or lower,
4. The therapeutic agent for Down's syndrome according to any one of items 1 to 3, wherein an age of the patient with the Down's syndrome is 36 or younger,
5. An agent for improving a daily activity of a Down's syndrome patient, comprising an acetylcholinesterase inhibitor,
6. The agent for improving the daily activity according to item 5, wherein the acetylcholinesterase inhibitor is donepezil or a pharmaceutically acceptable salt thereof.
7. The agent for improving the daily activity according to item 5 or 6, wherein an intelligence quotient of the patient with the Down's syndrome is 48 or lower,
8. The agent for improving the daily activity according to any one of items 5 to 7, wherein an age of the patient with the Down's syndrome is 36 or younger,
9. An agent for improving a mental retardation in Down's syndrome, comprising an acetylcholinesterase inhibitor,
10. The agent for improving the mental retardation in Down's syndrome according to item 9, wherein the acetylcholinesterase inhibitor is donepezil or a pharmaceutically acceptable salt thereof,
11. The agent for improving the mental retardation in Down's syndrome according to item 9 or 10, wherein an intelligence quotient of a patient with the Down's syndrome is 48 or lower,
12. The agent for improving the mental retardation in Down's syndrome according to any one of items 9 to 11, wherein an age of the patient with the Down's syndrome is 36 or younger,
13. An agent for mitigating or improving a symptom of rapid retrogression in Down's syndrome, comprising an acetylcholinesterase inhibitor,
14. The agent for mitigating or improving the symptom of rapid retrogression in Down's syndrome according to item 13, wherein the acetylcholinesterase inhibitor is donepezil or a pharmaceutically acceptable salt thereof,
15. The agent for mitigating or improving the symptom of rapid retrogression in Down's syndrome according to item 13 or 14, wherein an intelligence quotient of a patient with the Down's syndrome is 48 or lower,
16. The agent for mitigating or improving the symptom of rapid retrogression in Down's syndrome according to any one of items 13 to 15, wherein an age of the patient with the Down's syndrome is 36 or younger,
17. A method for treating Down's syndrome, comprising administering an acetylcholinesterase inhibitor to a patient with the Down's syndrome.
18. The method for treating Down's syndrome according to item 17, wherein the acetylcholinesterase inhibitor is donepezil or a pharmaceutically acceptable salt thereof,
19. A method for improving a mental retardation or improving a daily activity of a Down's syndrome patient, comprising administering an acetylcholinesterase inhibitor to a patient with Down's syndrome.
20. The method according to item 19, wherein the acetylcholinesterase inhibitor is donepezil or a pharmaceutically acceptable salt thereof,
21. A method for mitigating or improving symptoms of rapid retrogression in Down's syndrome, comprising administering an acetylcholinesterase inhibitor to a patient with Down's syndrome,
22. The method for mitigating or improving symptoms of rapid retrogression in Down's syndrome according to item 21, wherein the acetylcholinesterase inhibitor is donepezil or a pharmaceutically acceptable salt thereof,
23. A composition for the treatment of Down's syndrome, comprising donepezil or a pharmaceutically acceptable salt thereof,
24. A composition for the improvement of a daily activity of a Down's syndrome patient, comprising donepezil or a pharmaceutically acceptable salt thereof,
25. A composition for the improvement of a mental retardation in Down's syndrome, comprising donepezil or a pharmaceutically acceptable salt thereof,
26. A composition for mitigating or improving a symptom of rapid retrogression in Down's syndrome, comprising donepezil or a pharmaceutically acceptable salt thereof.

### Advantageous Effect of the Invention

According to the present invention, there is provides a therapeutic agent and a method of treatment for patients with Down's syndrome, which is a congenital genetic disease for which no fundamental method of treatment exists. With the present invention it is possible to realize an improvement in QOL in daily activities that are most important to patients with Down's syndrome and their families; for example, an improvement in daily activities in which the mental stability of the patient is either maintained or enhanced, and in which the performance of daily activities, communication, socialization and the like are also improved. Moreover, the present invention can improve mental retardation, which is one characteristic of Down's syndrome, or promote mental development. Therefore, the present invention not only can provide efficacy in the mental development and daily activities of the patients with Down's syndrome themselves, but it also can alleviate the burden on the families during the period of raising the patient to adulthood. Furthermore, by receiving therapy during this time, the patient can lead a stable social life from adolescence to adulthood. More specifically, the present invention is effective even in patients with an IQ of 50 or lower in whom rehabilitation effect cannot be expected to be effective, and the present invention improves daily activities of patients and facilitates their participation in social activities.

### Brief Description of Drawings

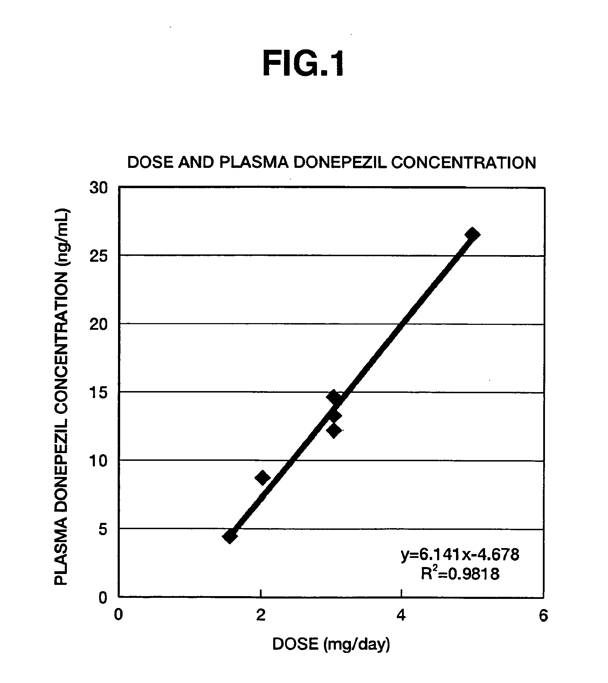

Figure 1 shows the changes in plasma donepezil concentration with respect to dose of donepezil hydrochloride.

### Best Mode for Carrying Out the Invention

The following embodiments are examples for explaining the present invention, but the present invention is in no way restricted to these embodiments. The present invention can be carried out by those skilled in the art using a variety of embodiments provided they do not depart from the scope and spirit of the invention.

The object of the present invention can be achieved by administering the acetylcholinesterase inhibitor, donepezil or a pharmaceutically acceptable salt thereof, or a pharmaceutical composition containing donepezil or a pharmaceutically acceptable salt thereof to a patient with Down's syndrome. In this context, the term "patient with Down's syndrome" refers to a patient who has been objectively diagnosed by a chromosome test and specialist physician and in whom congenital delayed psychomotor development is seen starting immediately after birth. Although variation is seen among individuals, delayed psychomotor development affects every range of daily activities such as movement, communication, cognition, social and interpersonal skills, personal independence and self management, autonomy, learning ability, and the like, and therefore participation by the patient in social and group activities becomes a problem.

In the present invention, the targets of administration of donepezil or a pharmaceutically acceptable salt thereof are not restricted to the following examples, but it is preferred that the patient be one that does not exhibit the behavioral symptoms of Alzheimer-type dementia until the early thirties, i.e., patients who are 36 or younger. In addition administration is preferred to patients younger than 20 in whom no decline in mental function due to Alzheimer's disease and the like has been seen.

In Down's syndrome various rehabilitative programs are performed from infancy under the supervision of physicians and occupational therapists. For example, guidance is provided for the motor development of the skilled motor control and fine motor control systems, language development such as language comprehension and expression, performance of daily activities, personal independent behavior and group activities, socialization and the like. It is also possible to combine pharmacotherapy using donepezil or a pharmaceutically acceptable salt thereof with rehabilitation. Moreover, with the present invention superior efficacy can be achieved in patients with an IQ of 50 or lower in whom sufficient efficacy could not be obtained in the past with rehabilitation.

The present invention enables the improvement of mental retardation, the promotion of mental development, and improvement in daily activities in patients with Down's syndrome. Confirmation of the improvement of mental retardation and promotion of mental development is made possible through an overall evaluation of patient behavioral changes in daily activities and changes in IQ. Patient behavioral changes in daily activities can be evaluated by objective observation or observational records kept by family members who live with the patient, physicians and rehabilitation_staff who supervise the patient, educational staff at school, persons in contact with the patient at work and the like. It is also possible to evaluate the patient's language functions such as sentence construction ability and expressiveness from the patient's diary or pictures drawn by the patient and the like. It is possible to evaluate the patient's mental state from the patient's interest in and recognition of others, from emotional changes, from sentences constructed by the patient and from the composition and colors used in pictures drawn by the patient and the like. The IQ can be evaluated by standard measurements performed during medical treatment or at an educational site and the like. However, the evaluation of the improvement of mental retardation and the improvement in daily activities in Down's syndrome is not limited to these assessment methods.

In the present invention, the term "improvement in daily activities" indicates an improvement, increase, or acceleration in the mental and emotional stability of the patient; in the performance of daily activities such as the ability to get ready by oneself, perform job skills and the like; in eagerness to learn and in exhibiting learning ability in an educational setting and at the workplace; in establishing rhythmic regularity in activities; in communication skills such as showing interest in and curiosity about others, language functions such as language comprehension, language expression and the like; in adaptiveness to group activities and autonomy in group activities; and in socialization such as showing personal initiative and the like. Moreover, with respect to the mental and emotional stability of the patient, the term "improvement in daily activities" includes the nearly complete disappearance of mentally unstable states (feelings of weakness, lack of motivation, irregular activities, inappropriate behavior, unsafe behavior) on a daily basis and the continuation of a stable state. In addition, the term "improvement in daily activities" includes improvement in physical condition such as bodily conditions that can be affected mentally, for example, sleep, menstrual cycle and bowel movements, a change in body weight to an appropriate body weight (i.e., dieting and the like), and resistance to infectious disease and the like. Therefore, in the present invention the agent for improving daily activities that contains donepezil hydrochloride is an agent that improves the aforementioned specific kinds of behavior and the like.

The donepezil or a pharmaceutically acceptable salt thereof in the present invention, for example, donepezil hydrochloride, can be manufactured by publicly known methods such as the method described in Japanese Patent No. 2578475, but the present invention places no restriction on the method of manufacture, and donepezil or a pharmaceutically acceptable salt thereof already on the market can be easily obtained commercially. Examples of the pharmaceutically acceptable salt in the present invention include, for example, the salts of inorganic acids, the salts organic acids and the like. Preferred examples of a salt-forming inorganic acid include hydrochloric acid, hydrobromic acid, sulfuric acid, nitric acid, phosphoric acid, and the like; preferred examples of a salt-forming organic acid include, for example, acetic acid, succinic acid, fumaric acid, maleic acid, tartaric acid, citric acid, lactic acid, stearic acid, benzoic acid, methanesulfonic acid, p-toluenesulfonic acid, and the like. The formula of donepezil hydrochloride is shown below.

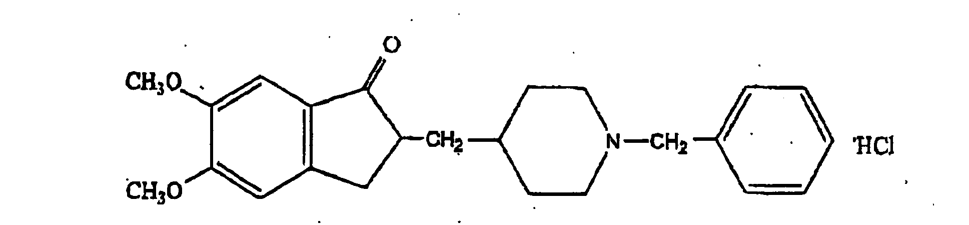

In the present invention, a pharmaceutical composition containing donepezil or a pharmaceutically acceptable salt thereof can manufactured by the conventional methods in the field of pharmaceutical formulation. For example, the present invention places no particular restriction on the dosage form or method of manufacture, and the pharmaceutical composition according to the present invention can be formulated as a liquid, semisolid, or solid. In other words, a medicine containing donepezil hydrochloride can be used by formulating it into an oral medication such as fine granules, tablets, capsules, tablet triturates, rapidly disintegrating tablets, liquids, suspensions, syrups and the like. In addition, as a parenteral agent, the pharmaceutical composition can be used in a dosage form such as injections, lyophilized agents, suppositories, patches and the like, although an oral dosage form is preferred. For example, the tablets or the fine granules that are currently used for the treatment of mild and moderate Alzheimer-type dementia (brand name: Aricept, Eisai Co., Ltd.) can be used.

Excipients, binders, disintegrating agents, lubricants, flavor enhancers, sweeteners, suspension-forming agents, emulsifiers, flavoring agents, coloring agents, antioxidants, gelling agents, stabilizers, pH regulators, antiseptics, preservatives, and the like can be used as additives to the pharmaceutical composition according to the present invention, and the present invention places no particular restriction on additives. Examples of excipients include, but are not limited to, lactose, D-mannitol, corn starch, crystalline cellulose, light anhydrous silicic acid, anhydrous calcium phosphate, precipitated calcium carbonate, calcium silicate, cocoa butter, liquid paraffin, soybean oil, olive oil, medium chain fatty acid triglycerides, glycerin, propylene glycol and the like. Examples of binders include, but are not limited to, polyvinyl pyrrolidone, dextrin, hydroxypropyl methylcellulose, methylcellulose, polyvinyl alcohol, carboxymethylcellulose sodium, partially alphatized starch, sodium alginate, pullulan, acacia gum powder and the like. Examples of disintegrating agents include, but are not limited to, low-substituted hydroxypropyl cellulose, carboxymethylcellulose sodium, crospovidone and the like. Examples of lubricants include, but are not limited to, sucrose-fatty acid esters, magnesium stearate, sodium stearyl fumarate, stearic acid, talc, Macrogol, and the like. Examples of flavor enhancers include, but are not limited to, citric acid, malic acid, monosodium glutamate, 5'-inosine monophosphate and the like. Examples of sweetens include, but are not limited to, glucose, fructose, sucrose, erythritol, maltitol, trehalose, sorbitol, xylitol, aspartame, acesulfame potassium, saccharine sodium, dipotassium glycyrrhizinate and the like. Examples of suspension-forming and emulsifying agents include, but are not limited to, lecithin, sucrose fatty acid esters, polyglycerin fatty acid esters, polyoxyethylene-hardened castor oil, polysorbate, polyoxyethylene/polyoxypropylene copolymer and the like. Examples of flavoring agents include, but are not limited to, menthol, peppermint oil, lemon oil, orange oil and the like. Examples of coloring agents include, but are not limited to, titanium oxide, red iron oxide, yellow iron oxide, sodium copper chlorophylline and the like. Examples of antioxidants include, but are not limited to, sodium ascorbate, L-cysteine, sodium sulfite, natural vitamin E and the like. Examples of gelling agents include, but are not limited to, carrageenan, pectin, sodium alginate, guar gum, locust bean gum, xanthan gum, carboxyvinyl polymer, carboxymethylcellulose, gelatin and the like. Examples of stabilizers include, but are not limited to, sodium polyphosphate, sodium metaphosphate and the like. Examples of pH regulators include, but are not limited to, citric acid, sodium citrate, hydrochloric acid, sodium hydroxide, disodium phosphate and the like. Examples of antiseptics and preservatives include, but are not limited to, sorbic acid, benzoate, paraben and the like.

The dose of donepezil or the pharmaceutically acceptable salt thereof in the present invention varies depending on severity of symptoms; differences in age, sex, body weight, and sensitivity; method of administration; time of administration, interval between doses, and the properties, formulation, and type of medicinal agent; type of active ingredients, and the like, and the present invention places no particular restrictions thereon. For example, just as in the dose for treating Alzheimer-type dementia, after administration for 1 to 2 weeks of a dose lower than the effective dose, the effective dose may be administered. The normal dose for adults is from about 0.05 to 100mg per day, preferably from about 0.1 to 10mg per day, and more preferably from about 0.5 to 5mg. Typically, this dose can be divided and administered 1 to 4 times a day. More specifically, when donepezil hydrochloride is administered repeatedly at a dose of 3mg/day, almost no adverse reactions are seen, and efficacy of the present invention can be expected. At a dose of 3mg/day if the concentration in the blood does not increase and sufficient efficacy does not appear, efficacy of the present invention can be expected by increasing the dose to 5mg/day, for example.

### Experimental Example 1

The followings are the results of a double blind study involving administration to patients with Down's syndrome. The double blind study was conducted for 24 weeks on the 14 patients with Down's syndrome shown in Table 1 who had no severe complications. The 7 patients in the experimental group who were treated with the active pharmaceutical ingredient were administered 1 tablet containing 3mg of donepezil hydrochloride in the first week of administration and 1 tablet containing either 5mg or 3mg of donepezil hydrochloride thereafter once a day after breakfast. The patients in the placebo group were administered 1 tablet of placebo corresponding to the medication containing 3mg of donepezil hydrochloride in the first week of administration, and 1 tablet of placebo corresponding to the medication containing 5mg of donepezil hydrochloride thereafter once a day after breakfast.

**Table 1 Patients with Down's syndrome in double blind study**

| | Subject No. | Sex | Age (yr) | IQ | Height (cm) | Weight (kg) |
|---|---|---|---|---|---|---|
| Experimental group | A | F | 36 | 48 | 143 | 54 |
| | B | F | 25 | 31 | 145 | 75 |
| | C | F | 36 | <20 | 139 | 53 |
| | D | M | 21 | 48 | 154 | 60 |
| | E | M | 34 | 30 | 152 | 42 |
| | F | M | 15 | 30 | 157 | 70 |
| | G | M | 21 | <20 | 151 | 52 |
| Placebo group | H | F | 25 | 41 | 150 | 57 |
| | | M | 15 | 37 | 158 | 44 |
| | J | M | 23 | 30 | 153 | 70 |
| | K | M | 20 | 30 | 160 | 49 |
| | L | M | 28 | 30 | 152 | 70 |
| | M | F | 15 | 35 | 140 | 48 |
| | N | M | 27 | 30 | 149 | 55 |

In the experimental group no adverse drug reactions were seen during administration of 3mg. Mild adverse reactions (diarrhea, nausea) were seen transiently in 4 of 7 patients when the dose was increased to 5mg. These symptoms disappeared when the dose was reduced to 3mg. Even after the dose was reduced to 3mg, 1 of the subjects complained of continuing gastrointestinal symptoms, and after a house visit by the physician, the patient was temporarily suspended from the study. After 6 months or more had passed, administration was started once more at 2mg, the dose was increased to 3mg starting 3months after the start of administration, and no adverse reactions were seen.

Improvement in daily activities was evaluated by house visits by the physician and by an interview questionnaire concerning the patient's daily activities completed at the time of the house visit by family members. During the administration period the patient and family visited the hospital once a month for a medical examination. Table 2 shows the results of the efficacy in improving daily activities of patients with Down's syndrome at the end of the study. In the experimental group some efficacy was seen in all subjects and more specifically, a ratio of the subjects in which the response was judged to be "excellent" was 57.1 % of the total. In the placebo group, the highest ratio of 57.1 % was seen in the subjects in which the response was judged to be "fair."

**Table 2 Comparison between the experimental group and the placebo group**

| | | Excellent | Good | Fair | Ineffective | Total |
|---|---|---|---|---|---|---|
| Experimental group | (persons) | 4 | 1 | 2 | 0 | 7 |
| | (%) | 57.1 | 14.3 | 28.6 | 0.0 | 100.0 |
| Placebo group | (persons) | 1 | 0 | 4 | 2 | 7 |
| | (%) | 14.3 | 0.0 | 57.1 | 28.6 | 100.0 |

In the cases of being judged "excellent" in the experimental group the family members living together with the patients every day recognized a clear improving effect, and that improving effect increased as the administration period lengthened. In addition, persons at work and acquaintances also recognized an improving effect in the performance of everyday activities. For example, the appearance and improvement of independent acts such as taking spontaneous action in getting out of bed and at meals, writing or drawing pictures spontaneously, commenting on the social column of the newspaper, using transportation facilities independently, and the like were seen. In addition, spontaneous, positive, voluntary behavior from the patient was seen concerning tasks at work and at home. Moreover, enhanced socialization such as improved energy and ability to work, interest in others, improved ability to respond on the telephone and the like were seen. Furthermore, an improvement in overall language functions such as improved language expressive functions including linguistic expressiveness, the number of vocabulary, and sentence-forming ability, and improved receptive language functions including the ability to comprehend meaningful words and the like were recognized. In patients in which administration was judged "effective," improvement in language expressive functions such as saying words they had never said before and the like, and an improving effect in the performance of daily activities such as a faster response in daily activity events. In cases that were judged to be "fair," efficacy in improving physical condition was recognized by a loss of weight and approach to appropriate body weight and the like through maintenance of a mentally stable condition and stable performance of daily activities during the administration period.

There was clearly no change in 2 of the subjects in the placebo group, and the family was convinced that the patient was receiving the placebo. In 4 cases in which the efficacy was judged "fair," there were instances in which efficacy was seen only in some items, or efficacy was seen but the effect was not stable and there was scattering in the results of the monthly assessments. For example, in 1 subject (age 15), an improvement in quality and quantity was seen in language functions, but no effect was seen in other adaptive behavior and ability to work.

The active pharmaceutical ingredient was administered to 5 of the 7 subjects in the placebo group after completion of the 6-month double blind study. Administration of the active pharmaceutical ingredient was performed using the same method used for the experimental group in the double blind study, but 1 of the 5 subjects had a decline in liver function values, so at 2 months a dose of 1.5mg/day was administered, another evaluation was performed at 4 months, and administration was discontinued. Table 3 shows a comparison of results between the placebo administration period and the active pharmaceutical ingredient administration period with respect to efficacy in improving daily activities in Down's syndrome patients. Whereas the response in 14.3% of the cases was judged "good" or better when they were administered the placebo, the response was judged "good" or better in 80.0% or more of the cases after they were administered the active pharmaceutical ingredient.

**Table 3 Comparison between administrations of the placebo and the active pharmaceutical ingredient**

| | | Excellent | Good | Fair | Ineffective | Total |
|---|---|---|---|---|---|---|
| Placebo | (Persons) | 1 | 0 | 4 | 2 | 7 |
| | (%) | 14.3 | 0.0 | 57.1 | 28.6 | 100.0 |
| Active pharmaceutical ingredient | (Persons) | 2 | 2 | 1 | 0 | 5 |
| | (%) | 40.0 | 40.0 | 20.0 | 0.0 | 100.0 |

In all subjects the evaluation results for administration of the active pharmaceutical ingredient were higher than the evaluation results for the placebo. For example, after administration of the active pharmaceutical ingredient, responses of "excellent" and "fair" were obtained in 2 cases in which the response had been judged "ineffective" when the placebo was administered. Among these, in the subject in which the response was judged "excellent" an increase in socialization such as showing personal initiative as revealed by expressing an opinion and the like, and in communication ability and the like was recognized, and an improving effect in language functions such as the number of vocabulary, ability to comprehend meaning, sentence-forming ability and the like were seen. In 2 cases in which the response was judged "fair" with administration of the placebo, the response improved to "good" with administration of the active pharmaceutical ingredient. Among these, in 1 subject the scores for personal initiative and group activities in the performance evaluation on the payslip at work improved for the first time. Among the cases of being judged "excellent" when they were administered the placebo and the active pharmaceutical ingredient, the improvement efficacy with the active pharmaceutical ingredient and the improvement efficacy with the placebo clearly differed qualitatively. In other words, whereas an improvement in language ability alone was seen with the placebo, an improving effect was seen in a plurality of parameters with the active pharmaceutical ingredient such as increased conversation ability, independent use of transportation facilities, improvement in arithmetic calculations and the like.

The blood concentration of the active pharmaceutical ingredient in each subject was measured 4 weeks after the start of administration of the active pharmaceutical ingredient during the above double blind study and during the administration period of the active pharmaceutical ingredient after the end of the double blind study. The blood concentration in 8 subjects (weight 42kg to 70kg, mean weight: 55.6kg) during administration of the 3mg dose of donepezil hydrochloride was 12.1 ng/mL to 23.0ng/mL, and the mean was 16.9ng/mL. The blood concentration in 3 subjects (weight 54kg to 75kg, mean weight: 66.3kg) during administration of the 5mg dose of donepezil hydrochloride was 26.3ng/mL to 26.6ng/mL, and the mean was 26.4ng/mL. In addition, among the subjects in which the dose was changed the blood concentration at different doses was measured in 3 subjects, and the correlation between the two doses was found (Figure 1). Furthermore, almost no adverse reactions occurred in any of the subjects when the blood concentration of donepezil hydrochloride was 23ng/mL or less and the dose was 3mg/day of donepezil hydrochloride.

### Experimental Example 2: Administration to patients with rapid retrogression

### (Method of Administration)

In the following patient "symptoms of acute retrogression" were confirmed as a result of examination by a physician and interview questionnaire concerning the daily activities. After confirmation, oral administration of 2mg/day of donepezil hydrochloride was initiated, and changes in symptoms were confirmed by house visits once a month.
Patient: The patient was 12 years, 5 months old and had an IQ of 47. The vocabulary age was 4 years, 2 months (determined by a picture vocabulary development test). The social activity age was 4 years, 6 months (determined by a social activity ability test). The patient exhibited no symptoms of dementia (evaluated by dementia scale for Down's syndrome patients). Retrogression was present (determined by survey questionnaire concerning retrogression).

### (Study Results)

### House visit after 1 month

The situation of the patient was confirmed 1 month after the start of administration. With respect to the daily activities, before administration the patient required 30minutes or longer to dress and undress, but after administration these acts could be performed in 2 to 3 minutes. Before administration the patient's actions to move from one place to another were sluggish, and the patient could not move unless a companion called out to the patient, but after administration the patient could move from one place to another spontaneously. Swaying of the body had been present, but after administration the patient exhibited no swaying in daily activities unless listening to music. In the aspect of communication as well, the patient started to begin conversations spontaneously by asking permission and the like. The patient began to eat more beginning 2 weeks after the start of administration, and began to live an orderly life such as spontaneously getting out of bed in the morning and the like. No severe adverse reactions were seen.

### House visit after 2 months

It was confirmed that the subject was spontaneously getting up in the morning with extreme regularity. In addition, in daily activities the patient was sorting laundry, and before administration could not fold one piece in 30minutes, but after administration could process 10 to 20 pieces in 10minutes, and was approaching the level prior to the symptoms of rapid retrogression. Furthermore, the patient was able to spontaneously take medicine without the instruction of a parent. In addition, the adverse reactions such as gastrointestinal symptoms, panic, decrease in muscle tone and the like disappeared entirely during the administration period. The blood concentration was 13.7ng/mL.

### House visit after 3 months

It was confirmed that the improving effects observed in the 1 and 2 month house visits were continuing. In this study an improvement in the behavioral aspects of the symptoms of rapid retrogression were seen in the short period of 3 months. Efficacy was seen in the improvement of performance of daily activities and problematic behavior.

### Industrial Applicability

In accordance with the present invention a therapeutic agent for Down's syndrome and a method for treating the same are provided by the administration of donepezil hydrochloride, and daily activities of patients with Down's syndrome are improved.

## Claims

1. A therapeutic agent for Down's syndrome, comprising an acetylcholinesterase inhibitor.

2. The therapeutic agent for Down's syndrome according to claim 1, wherein the acetylcholinesterase inhibitor is donepezil or a pharmaceutically acceptable salt thereof.

3. The therapeutic agent for Down's syndrome according to claim 1 or 2, wherein an intelligence quotient of a patient with Down's syndrome is 48 or lower.

4. The therapeutic agent for Down's syndrome according to any one of claims 1 to 3, wherein an age of the patient with the Down's syndrome is 36 or younger.

5. An agent for improving a daily activity of a Down's syndrome patient, comprising an acetylcholinesterase inhibitor.

6. The agent for improving the daily activity according to claim 5, wherein the acetylcholinesterase inhibitor is donepezil or a pharmaceutically acceptable salt thereof.

7. The agent for improving the daily activity according to claim 5 or 6, wherein an intelligence quotient of the patient with the Down's syndrome is 48 or lower.

8. The agent for improving the daily activity according to any one of claims 5 to 7, wherein an age of the patient with the Down's syndrome is 36 or younger.

9. An agent for improving a mental retardation in Down's syndrome, comprising an acetylcholinesterase inhibitor.

10. The agent for improving the mental retardation in Down's syndrome according to claim 9, wherein the acetylcholinesterase inhibitor is donepezil or a pharmaceutically acceptable salt thereof.

11. The agent for improving the mental retardation in Down's syndrome according to claim 9 or 10, wherein an intelligence quotient of a patient with the Down's syndrome is 48 or lower.

12. The agent for improving the mental retardation in Down's syndrome according to any one of claims 9 to 11, wherein an age of the patient with the Down's syndrome is 36 or younger.

13. An agent for mitigating or improving a symptom of rapid retrogression in Down's syndrome, comprising an acetylcholinesterase inhibitor.

14. The agent for mitigating or improving the symptom of rapid retrogression in Down's syndrome according to claim 13, wherein the acetylcholinesterase inhibitor is donepezil or a pharmaceutically acceptable salt thereof.

15. The agent for mitigating or improving the symptom of rapid retrogression in Down's syndrome according to claim 13 or 14, wherein an intelligence quotient of a patient with the Down's syndrome is 48 or lower.

16. The agent for mitigating or improving the symptom of rapid retrogression in Down's syndrome according to any one of claims 13 to 15, wherein an age of the patient with the Down's syndrome is 36 or younger.

17. A method for treating Down's syndrome, comprising administering an acetylcholinesterase inhibitor to a patient with the Down's syndrome.

18. The method for treating Down's syndrome according to claim 17, wherein the acetylcholinesterase inhibitor is donepezil or a pharmaceutically acceptable salt thereof.

19. A method for improving a mental retardation or improving a daily activity of a Down's syndrome patient, comprising administering an acetylcholinesterase inhibitor to a patient with Down's syndrome.

20. The method according to claim 19, wherein the acetylcholinesterase inhibitor is donepezil or a pharmaceutically acceptable salt thereof.

21. A method for mitigating or improving symptoms of rapid retrogression in Down's syndrome, comprising administering an acetylcholinesterase inhibitor to a patient with Down's syndrome.

22. The method for mitigating or improving symptoms of rapid retrogression in Down's syndrome according to claim 21, wherein the acetylcholinesterase inhibitor is donepezil or a pharmaceutically acceptable salt thereof.

23. A composition for the treatment of Down's syndrome, comprising donepezil or a pharmaceutically acceptable salt thereof.

24. A composition for the improvement of a daily activity of a Down's syndrome patient, comprising donepezil or a pharmaceutically acceptable salt thereof.

25. A composition for the improvement of a mental retardation in Down's syndrome, comprising donepezil or a pharmaceutically acceptable salt thereof.

26. A composition for mitigating or improving a symptom of rapid retrogression in Down's syndrome, comprising donepezil or a pharmaceutically acceptable salt thereof.
